# EUROPEAN PATENT APPLICATION

(11) **EP 4 194 444 A1**
(43) Date of publication of application: **14.06.2023**
(21) Application number: 21852960.0
(22) Date of filing: 05.08.2021
(51) Int. Cl.: C07D 263/60, C07D 493/10, G01N 21/78, G01N 31/22, G01N 33/14, G01N 33/493, C07C 59/01

(54) **NOVEL BENZOXAZOLE DERIVATIVES, METHOD FOR PRODUCING SAME AND USE THEREOF TO DETECT GHB IN DRINKS AND URINE**

(30) Priority: 06.08.2020 ES 202030840
(71) Applicant: Universitat de València, 46010 Valencia (ES)
(72) Inventor: RODRIGUEZ NUÉVALOS, Silvia, 46010 VALENCIA (ES); COSTERO NIETO, Ana María, 46010 VALENCIA (ES); PARRA ÁLVAREZ, Margarita, 46010 VALENCIA (ES); GIL GRAU, Salvador, 46010 VALENCIA (ES); GAVIÑA COSTERO, Pablo, 46010 VALENCIA (ES); HERNANDEZ CONTRERAS, Jordi, 46010 VALENCIA (ES)
(74) Representative: Moreno Sares, Rafael
(86) International application number: PCT/ES2021/070595
(87) International publication number: WO 2022/029357

(57) **Abstract**

The present invention relates to compound 1 or 2 or the composition comprising it. The present invention also relates to a process for obtaining compound 1 or 2, as well as to the use thereof for detecting GHB in beverages and urine. An equipment or kit for use in detecting GHB in beverages and urine is also included.

## Description

### Field of the invention

The present invention relates to new benzoxazole-based compounds, to a process for obtaining them and to their use in the detection of γ-hydroxybutyric acid (GHB) in beverages and urine.

### Background

γ-hydroxy butyric acid (GHB) is one of the drugs used in chemical submission. It is an odorless, colorless, slightly salty compound sometimes used for recreational purposes. However, this compound, when supplied without knowledge of the victim, has been used on numerous occasions in order to commit an offense. In many of them, it is used to void the will of the victims and carry out sexual assault. In general, the intake of the drug by the victim occurs through a beverage that has been contaminated with the product without noticing the product. Residence time in the body is 3-6 hours and its metabolites are excreted rapidly, so that it is very difficult to detect their presence in the body after the assault.

Four references addressing this subject have been found in the scientific literature (J. Forensic. Sci. 2004, 49, p. 379-387, Chem. Commun. 2013, 49, p. 6170-6172, Chem. Commun. 2014, 50, p. 2904-2906, J. Mat. Chem. B 2017, 8, p. 2736-2742).

The first case (J. Forensic. Sci. 2004, 49, p. 379-387) relates to an enzymatic reaction while the other three reviews are referring to chemical sensors.

Chem. Commun. 2014, 50, 2904-2906 describes the use of Compound A as chemosensor for detecting GHB. The interaction with the analyte was based on a hydrogen bonding interaction between the phenol group on the sensor and the carboxylate unit on GHB.

In addition, the same authors also described in Chem. Commun. 2013, 49, 6170- 6172 the use of Compound B for detecting the corresponding lactone (GBL), but again, the interaction was related to the formation of a hydrogen bond. However, in any case, soft drinks alone or mixed with alcoholic beverages were not tested as interferents.

On the other hand, J. Mat. Chem. B 2017, 8, 2736-2742 describes the preparation of a complex of Ir (C) capable of detecting GHB. The complex showed luminescence quenching in the presence of analyte that was observed using UV light.

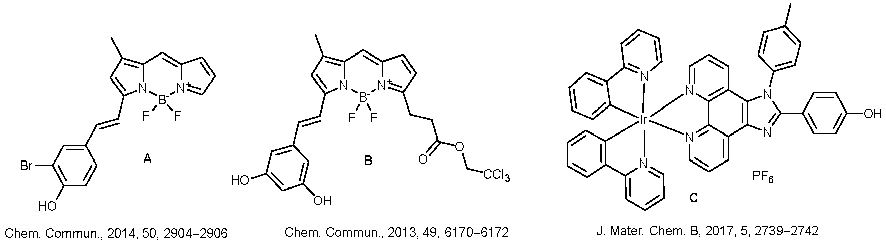

By following a different approach, Bravo D. T.; Harris, D. O., Parsons S. M. J. Forensic. Sci. 2004, 49, p. 379-387 described the use of γ-hydroxybutyrate dehydrogenase (GHB-DH) to detect GHB. Enzymatic oxidation of GHB by NAD⁺ is combined with reduction of a dye showing a colour change. Ethanol is an interferent that can be avoided by drying the sample to be studied.

The present inventors have been able to develop a reliable and easy-to-use procedure and device that enables visual determination of whether a beverage, whether alcoholic, non-alcoholic, or combined, has been contaminated with GHB prior to ingestion. Additionally, the ready-to-use device only needs a drop of the beverage under analysis so that in case the response is negative, it can be ingested substantially entirely without any problems. Likewise the present inventors have applied the same process for detecting GHB in urine.

### Summary of the Invention

In a first aspect, the present invention relates to a compound 1 or 2 according to claim 1.

In a second aspect, the present invention also relates to a composition comprising a compound 1 or 2 according to the first aspect.

In a third aspect, the present invention also relates to a process for obtaining a compound 1 or 2 according to the first aspect.

In a fourth aspect, the present invention relates to the use of a compound 1 or 2 according to the first aspect or a composition according to the second aspect for the detection of GHB in beverages or urine.

In a fifth aspect, the present invention relates to a procedure for detecting GHB in beverages.

In a sixth aspect, the present invention relates to a procedure for detecting GHB in urine.

In a seventh aspect, the present invention relates to an equipment or kit for use in detecting GHB in beverages and/or urine.

### Brief description of the drawings

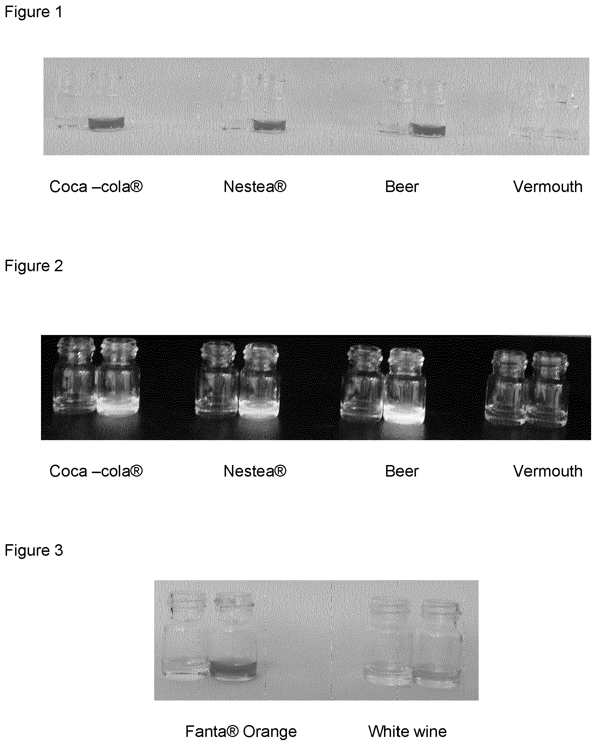

Figures 1 and 2 show the results of the real test with coca-cola0, nestea^{®}, beer and vermouth beverages with compounds 1 and 2, respectively. The color change in compound 1 and an increase in fluorescence in compound 2 are observed when adding the aliquot of contaminated beverage plus the weak base over the solution of compounds 1 or 2. The GHB in the beveage is at a concentration of 12 mM. After treatment with the base and mixing with the solution of compound 1 or 2 it is 0.18 mM. In the case of vermouth, the limitations in photography do not allow the difference to be seen so clearly. However, the inventors note that this difference is observable in vivo and would probably be observed even better at higher concentrations of GHB that would correspond to those used for criminal purposes.

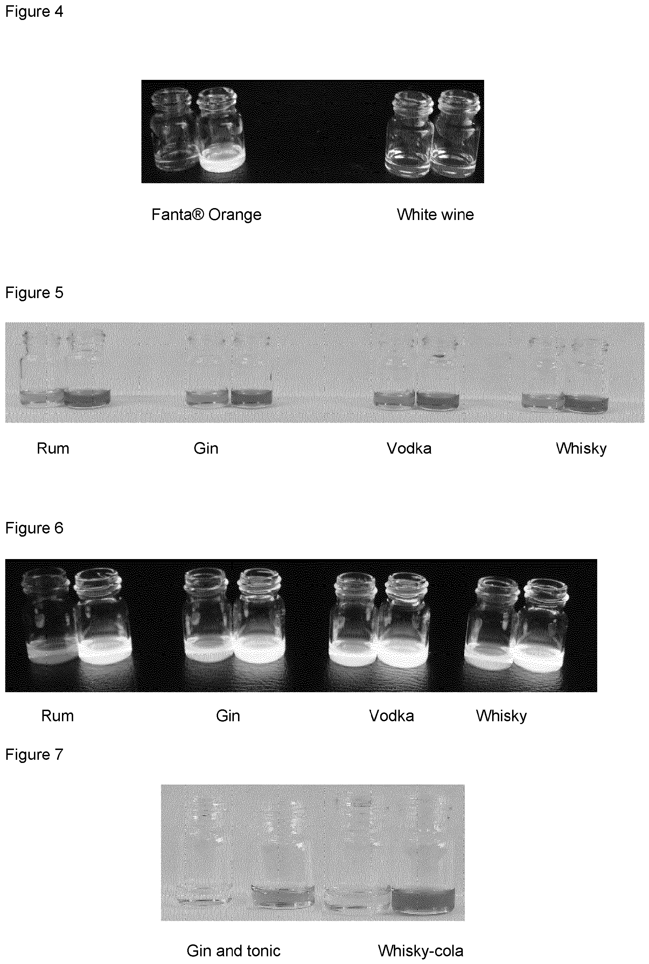

Figures 3 and 4 show the results of the real test with orange fanta^{®} and white wine beverages with compounds 1 and 2, respectively. The color change in compound 1 and an increase in fluorescence in compound 2 are observed when adding the aliquot of contaminated beverage plus the weak base over the solution of compounds 1 or 2. The GHB in the beverage is at a concentration of 12 mM. After treatment with the base and mixing with the sensor solution of compound 1 or 2 it is 0.18 mM. In the case of figure 4 for white wine, photography limitations do not allow the difference to be seen so clearly. However, the inventors note that this difference is observable in vivo and would probably be observed even better at higher concentrations of GHB that would correspond to those used for criminal purposes.

Figures 5 and 6 show the results from the real assay with rum, gin, vodka and whisky beverages with compounds 1 and 2, respectively. The color change in compound 1 and an increase in fluorescence in compound 2 are observed by adding the aliquot of contaminated beverage plus weak base over the solution of compound 1 or 2. GHB in the beverage is at a concentration of 12 mM. After treatment with base and mixing with dissolution of compound 1 or 2, it is 0.18 mM.

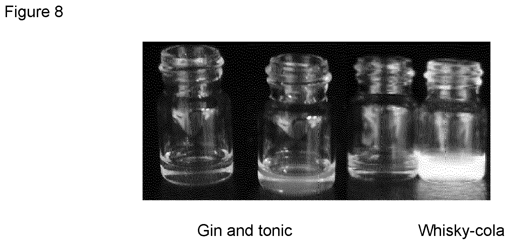

Figures 7 and 8 show the results from the real assay with gin-tonic and whisky-cola beverages with compounds 1 and 2, respectively. The color change in compound 1 and an increase in fluorescence in compound 2 are observed by adding the aliquot of contaminated beverage plus weak base over the solution of compound 1 or 2. GHB in the beverage is at a concentration of 12 mM. After treatment with base and mixing with dissolution of compound 1 or 2, it is 0.18 mM.

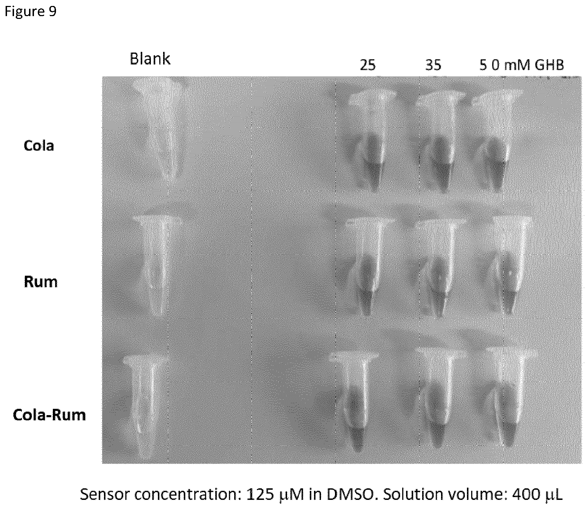

Figure 9 shows blank vials and vials with GHB concentrations of 25, 35, and 50 mM for cola, rum, and a combination of cola and rum using a compound 1 concentration of 125 µM.

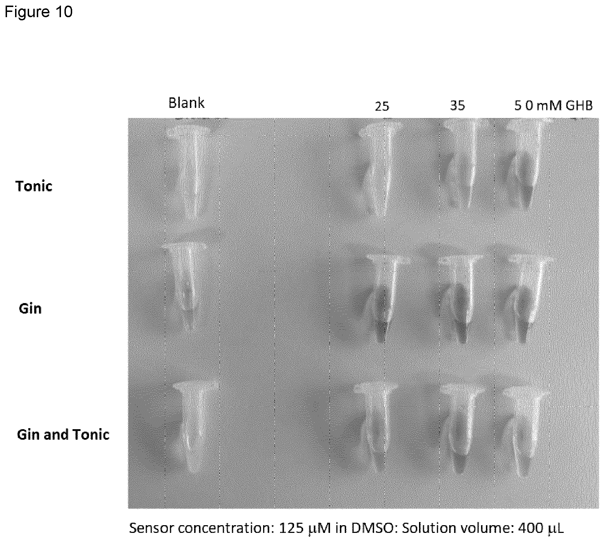

Figure 10 shows blank vials and vials with GHB concentrations of 25, 35, and 50 mM for tonic, gin, and a combination of tonic and gin using a compound 1 concentration of 125 µM.

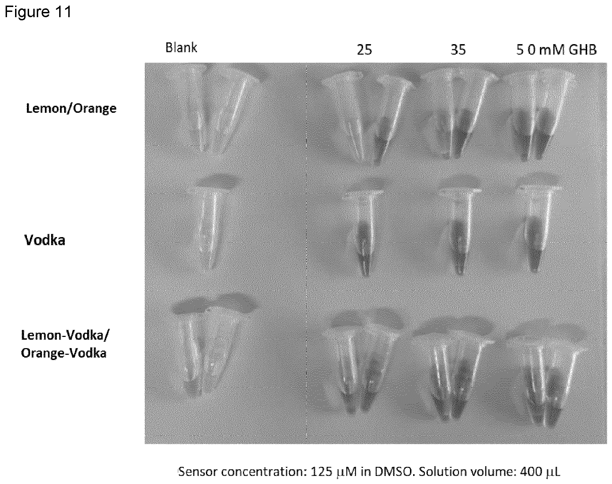

Figure 11 shows blank vials and vials with GHB concentrations of 25, 35, and 50 mM for lemon/orange soft drink, vodka, and a combination of lemon/orange soft drink and vodka using a compound 1 concentration of 125 µM.

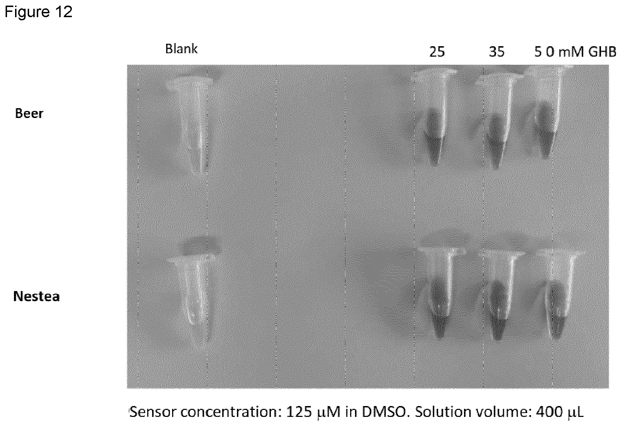

Figure 12 shows blank vials and vials with GHB concentrations of 25, 35, and 50 mM for beer and nestea^{®} using a compound 1 concentration of 125 µM.

### Detailed description of the invention

In a first aspect, the present invention relates to a compound of formula: 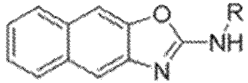 wherein R is

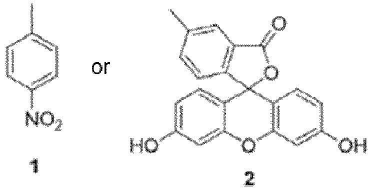

In a second aspect, the present invention relates to a composition comprising compound 1 or 2 as defined above. In a preferred embodiment, said composition comprises or consists of compound 1 or 2, as defined above, and an organic solvent. DMF (dimethylformamide) and DMSO (dimethylsulfoxide) may be used as the organic solvent, although DMSO is preferably used.

In a third aspect, the present invention relates to a process for obtaining compound 1, wherein R is 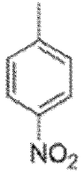 comprising the step:

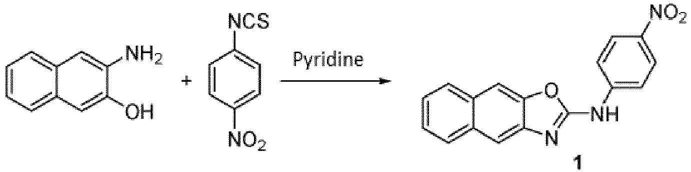

The reaction conditions are not particularly relevant as it can be carried out at room P and T, although an increase in temperature would likely increase the reaction rate.

The present invention also relates to a process for obtaining compound 2, wherein R is 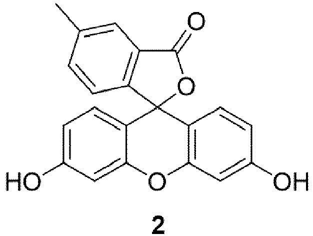 comprising the step:

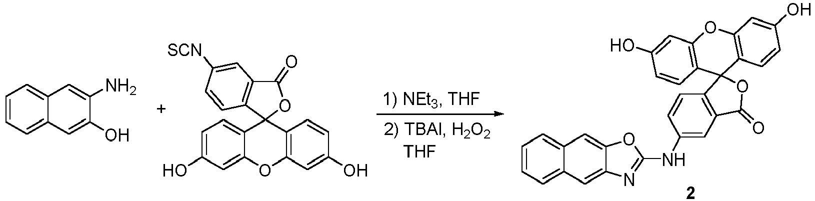

The reaction conditions are not particularly relevant as it can be carried out at room P and T.

In a fourth aspect, the present invention relates to the use of a composition, as defined above, or compound 1 or 2, as defined above, in the detection of γ-hydroxybutyric acid (GHB) or a salt thereof in beverages. Such salts may include, but are not limited to, salts with alkali or alkaline earth metals, preferably sodium. In the present invention, when reference is made to beverages, as indicated below, it will be understood as alcoholic beverages, non-alcoholic beverages or mixtures thereof. When reference is made to alcoholic beverages, alcoholic distillates such as, for example, whiskey or gin, as well as alcoholic beverages that are not distilled, such as wine and beer, are included.

The present invention relates to the use of a composition, as defined above, or compound 1 or 2, as defined above, in the detection of γ-hydroxybutyric acid (GHB) or a salt thereof in urine.

In a fifth aspect, the present invention relates to a process for detecting GHB or a salt thereof in beverages, comprising:
a) taking a drop from the beverage to be analyzed and dissolving it in an aqueous solution of weak base;
b) adding an aliquot of the solution obtained in step a) to the composition, as defined above, or alternatively, to compound 1 or 2, as defined above;
c) observing the reaction produced; and
d) qualitatively determining the presence of GHB in the beverage to be analyzed according to the observation of step c).

It should be noted that in step b) the composition of compound 1 or 2 and the organic solvent may already be prepared or may be prepared at that point of time just before the addition of the aliquot of the solution obtained in step a).

In a preferred embodiment, said weak base in step a) is sodium bicarbonate, although any weak base such as, for example, potassium, ammonium, calcium bicarbonate, could be used. In another preferred embodiment, the concentration of the aqueous solution of weak base is from 0.4 mM to 1 mM, most preferably 0.4 mM.

In another preferred embodiment, compound 1 or 2 is at a concentration from 50 µM to 500 µM, preferably from 50 µM to 100 µM.

In step c) it should be observed if a reaction occurs between compounds 1 or 2, alone or in the composition, with the possible GHB component or a salt thereof present in the beverage to be analyzed. If compound 1 reacts positively with GHB or a salt thereof, a color change (from yellow to red) will be observed, while if compound 2 reacts positively with GHB or a salt thereof, fluorescence will be observed (from dim green to intense yellow in distilled alcoholic beverages and from blue to green in combined beverages, soft drinks, or undistilled alcoholic beverages, such as beer, wine, or vermouth). Thus, one can conclude in step d) about the presence or not of GHB in the beverage being analyzed.

In another alternative embodiment of the process, this may be slightly modified in the order of mixing, although the final result would be the same. In this regard, the alternative process comprises the steps of:
a) mixing an aqueous solution of a weak base as defined above with the organic solvent as defined above;
b) adding compound 1 or 2 as defined above;
c) immediately after step b), adding a drop of the beverage to be analyzed;
d) observing the reaction produced; and
e) qualitatively determining the presence of GHB in the beverage to be analyzed according to the observation of step d).

In another alternative embodiment of the process, the visual detection is performed on a strip of a non-woven fabric (NWF) with 50 to 100% polypropylene, preferably with 100% polypropylene. In this case, the process for detecting GHB or a salt thereof in beverages comprises the steps of:
a) introducing a strip of non-woven fabric containing at least 50% polypropylene into a beverage sample, typically between 3 and 5 seconds is enough;
b) introducing the beverage-impregnated strip of step a) into a container containing the composition of the invention, as defined above, or alternatively compound 1 or 2, as defined above, preferably the composition;
c) observing the reaction produced in the container; and
d) qualitatively determining the presence of GHB in the beverage to be analyzed according to the observation of step c).

Compound 1 or 2 is preferably at a concentration of 50 µM to 500 µM, preferably 50 µM to 100 µM.

The reaction is considered positive in GHB if in step c) a colorless to red color change is observed with compound 1 and an appearance of fluorescence is observed with compound 2.

This detection process with a fabric strip detects GHB in all types of pure beverages (alcoholic and non-alcoholic) and combined alcoholic and non-alcoholic beverages provided that it is at a concentration above 35 µM.

In a sixth aspect, the present invention relates to a process for detecting GHB or a salt thereof in urine, comprising:
a) preparing a urine sample from a subject by passing it through a chromatographic column, preferably column C18, washing with methanol and water, and subsequently lyophillizing and redissolving the sample with water,
b) preparing a mixture of an aqueous solution of a weak base and the composition as defined above, or alternatively compound 1 or 2 as defined above;
c) mixing the urine sample obtained in step a) with the mixture of step b);
d) diluting the mixture obtained in step c) with an organic solvent;
e) observing the reaction produced; and
f) qualitatively and/or quantitatively determining the presence of GHB in the urine to be analyzed according to the observation of step e).

To obtain positive results the urine sample should be analyzed between 0 and 3 hours after drinking the beverage.

In a preferred embodiment, said weak base in step b) is sodium bicarbonate, although any weak base such as, for example, potassium, ammonium, calcium bicarbonate could be used. In another preferred embodiment, the concentration of the aqueous solution of weak base is from 0.4 mM to 1.2 mM.

In another preferred embodiment, compound 1 or 2 is at a concentration of 0.5 mM to 1.5 mM, preferably 1 mM.

In step e) it should be observed if a reaction occurs between compounds 1 or 2, alone or in the composition, with the possible GHB component or a salt thereof present in the urine sample to be analyzed. If compound 1 reacts positively with GHB or a salt thereof, a color change (from yellow to red) will be observed, while if compound 2 reacts positively with GHB or a salt thereof, fluorescence will be observed. Thus, one can conclude in step f) about the presence or not of GHB in the beverage being analyzed. If one wishes to quantitatively know the amount of GHB present in the sample, e.g., UV spectroscopy may be applied.

DMF (dimethylformamide) and DMSO (dimethylsulfoxide) may be used as, but not limited to, the organic solvent for step d), although DMSO is preferably used.

In a seventh aspect, the present invention relates to a kit or equipment for use in detecting GHB or a salt thereof in beverages and/or urine comprising:
- a transparent container with opening and closure containing a composition as defined above, alternatively a compound 1 or 2, as defined above, optionally wherein said container containing compound 1 or 2 is accompanied by another container with the same characteristics but with organic solvent as defined above, i.e. DMF (dimethylformamide) or DMSO (dimethylsulfoxide) are preferably used, more preferably DMSO.
- a transparent container with opening and closure containing a weak base in the form of an aqueous solution;
- a tool for collecting a sample of the beverage to be analyzed

In another embodiment, in the case of detecting GHB or a salt thereof in beverages by means of a strip of a non-woven fabric (NWF) with 50 to 100% polypropylene, the kit or equipment alternatively comprises:
- a strip of non-woven fabric (NWF) with 50 to 100% polypropylene;
- a transparent container with opening and closure containing a composition as defined above, alternatively a compound 1 or 2 as defined above, optionally wherein said container containing compound 1 or 2 is accompanied by another container with the same characteristics but with organic solvent as defined above, i.e. DMF (dimethylformamide) or DMSO (dimethylsulfoxide) are preferably used, more preferably DMSO.

Optionally, such a kit or equipment according to any of the preceding embodiments (whether based on strips for detection or not) further comprises instructions for use.

As noted above herein, the weak base used for kit for beverages without strip detection and/or urine is preferably sodium bicarbonate, although any weak base such as potassium bicarbonate, ammonium bicarbonate, calcium bicarbonate could be used. In another preferred embodiment, the concentration of the aqueous solution of weak base when detecting beverages is 0.4-1 mM, even more preferably 0.4 mM. In the case of a urine sample, the concentration of the aqueous solution of weak base is preferably from 0.4 mM to 1.2 mM

Additionally, as noted herein above, in the case of beverage detection for any of the embodiments of the kit (whether based on strips for detection or not), compound 1 or 2 is preferably present in the composition at a concentration from 50 µM to 500 µM, preferably from 50 µM to 150 µM, more preferably from 50 µM to 100 µM. In the case of a urine sample, compound 1 or 2 is preferably present at a concentration from 0.5 mM to 1.5 mM, preferably 1 mM.

In a preferred embodiment, such transparent containers with opening and closure, according to any of the embodiments of the kit (whether based on strips for detection or not), are microtubes, graduated or not graduated, of polypropylene (e.g., Eppendorf^{™}).

In another preferred embodiment of the non-strip based kit, said tool for collecting the sample can be a dropper or a small spoon. In the case of the strip-based kit, said tool is not necessary.

In a more preferred embodiment, in the use, process, or kit or equipment, as defined in the foregoing aspects, the beverage is an alcoholic beverage. In another more preferred embodiment, said alcoholic beverage is admixed with a non-alcoholic beverage, preferably a soft drink or juice. In another more preferred embodiment, said beverage is only a non-alcoholic beverage, preferably a soft drink or a juice.

Preferably, said alcoholic beverage is selected from, by way of non-limiting example, gin, vodka, rum, whisky, white wine, vermouth, and beer.

Preferably, said soft drink or juice is selected from, by way of non-limiting example, tonic, orange soft drink, lemon soft drink, cola drink, tea drink and any fruit juice.

It should be noted that any combination of previous embodiments or instances or examples encompassed by the present invention is understood by one skilled in the art as being feasible in the context of the present invention.

The various particular instances or examples recited above, as well as the following examples, are provided for illustrative purposes only and are not intended to limit the scope of the present invention in any way.

### EXAMPLES

### Example 1. Synthesis of Compound 1

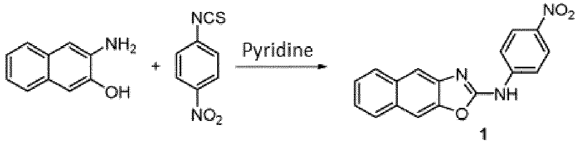

In a 50 L two-neck round-bottom flask, under inert atmosphere, 202 mg of 3-amino-2-naphthol (1.256 mmol) were dissolved along with 191 mg of 4-nitrophenyl isothiocyanate (1.047 mmol) in 13 mL of pyridine. Stirring was kept for 8 h at room temperature. The solvent was then evaporated, the solid dissolved in 35 mL of AcOEt and washed once with acidic water (about pH 5), once with NaHCO₃ (sat) and once with NaCl (sat). The organic phase was dried over MgSO₄, filtered and the solvent evaporated in vacuo. The crude was purified by silica-gel chromatography column using as eluent a 6:4 Hexane:AcOEt mixture. A slightly-orange yellow solid (Compound 1) was obtained in 35% yield.

### Example 2. Synthesis of Compound 2

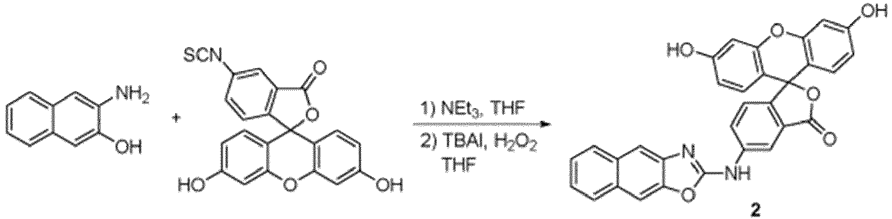

In a 25 mL one-neck round-bottomed flask, 45 mg of 3-amino-2-naphthol (0.283 mmol) were dissolved along with 98 mg of fluorescein-5-thioisocyanate (0.283 mmol) in 3 mL of THF. 50 µL of Net₃ were then added and allowed to stir at room temperature for 16 h. 34 µL of 30% H₂O₂ (0.565 mmol) and 1 mg of tetrabutylammonium iodide (0.003 mmol) were then added. An orange solid (Compound 2) was obtained in 79% yield.

### Example 3. Assays on real beverage samples

Initially, beverages are contaminated with GHB at a concentration of 12 mM. 100 µL of beverage are then taken and mixed with 100 µL of NaHCOs (1 mM or 0.4 mM). 30 µL of this solution were then mixed with 50 µL of sensor (from a 1 mM solution, in DMSO), previously dissolved in 920 µL of DMSO. The same process was followed for samples without GHB.

The results with compound 1 and compound 2 are seen in Figure 1 and Figure 2, respectively.

The results of a new assay with compound 1 and 2 on fanta^{®} orange beverages and white wine are seen in Figures 3 and 4, respectively.

The assay was applied again with compound 1 and 2 but with the following beverages: rum, gin, vodka and whisky. The results of the color changes are seen in Figures 5 and 6, respectively.

Finally, the assay with compounds 1 and 2 was applied to the gin and tonic and whisky-cola beverage. The results are seen in Figures 7 and 8, respectively.

### Example 4. Assay over urine sample

### Urine Sample Preparation

A C18 column (ExtraBond Cartridge C18 500 mg, 2 ml (Scharlau C18500- 03 L)) was used through which 1 mL of methanol and 1 mL of water were initially passed and discarded. 5 mL of a urine sample from the subject was then passed and collected in a petri dish. The column was washed first with 3 mL of a 5% methanol solution in deionized water followed by 3 mL of methanol. Both wash liquors were collected in the same Petri dish where the urine sample had been collected.

The solution was lyophilized to dryness and re-dissolved in 1 mL of water.

### Detection and measurement of GHB in the sample

10 µL of the prepared urine was mixed with 10 µL of a 1 mM NaHCOs aqueous solution and 100 µL of a 1 mM sensor solution. The mixture was diluted to completion of 3 mL with DMSO. The color change was observable to the naked eye or more accurately by UV spectroscopy, which allowed to further quantify the amount of GHB present in the urine sample.

### Example 5. GHB detection in beverages by polypropylene strip

A strip of a 100% polypropylene non-woven fabric (NWF) of 0.5 x 3.0 cm was introduced into the beverage for three seconds and then brought to a vial containing a 125 µM concentration sensor solution in DMSO. Upon contact of the strip with the sensor, a color change into red occurred in the case of compound 1 and strong fluorescence appeared in the case of compound 2. The system detects all types of combinations provided that the concentration is above 35 µM.

## Claims

1. A compound of formula: 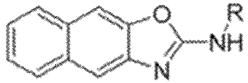 wherein R is

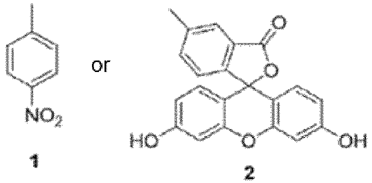

2. A composition comprising the compound according to Claim 1.

3. The composition according to claim 2, comprising the compound according to Claim 1 and an organic solvent.

4. The composition according to claim 2, consisting of the compound according to claim 1 and an organic solvent.

5. Composition according to claim 3 or 4, wherein the solvent is dimethylsulfoxide (DMSO).

6. A process for obtaining the compound according to claim 1 wherein R is 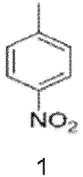 comprising the step:

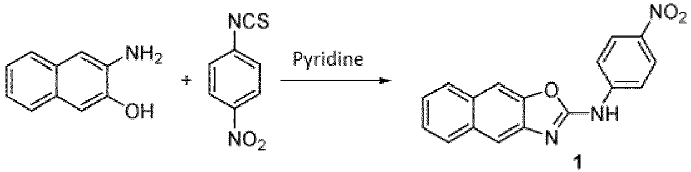

7. A process for obtaining the compound according to claim 1 wherein R is 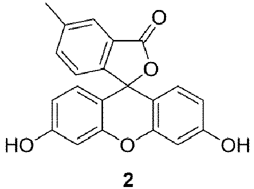 comprising the step:

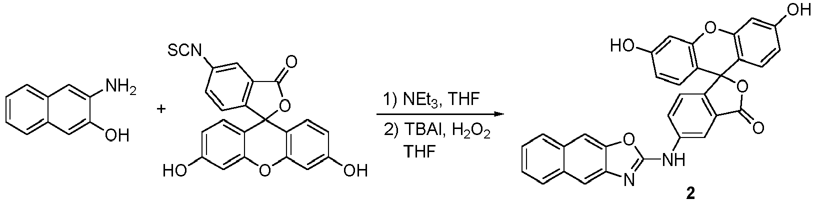

8. Use of a compound according to Claim 1 or a composition according to any of Claims 2 to 5 in the detection of γ-hydroxybutyric acid (GHB) or a salt thereof in beverages.

9. The use of a compound or composition according to Claim 8, wherein the beverage is an alcoholic beverage alone or mixed with a non-alcoholic beverage.

10. The use of a compound or composition according to Claim 8 wherein the beverage is a non-alcoholic beverage alone.

11. The use of a compound or composition according to claim 9 wherein the alcoholic beverage is selected from gin, vodka, rum, whisky, white wine, vermouth and beer.

12. The use of a compound or composition according to any of Claims 9 to 11 wherein the non-alcoholic beverage is a soft drink or juice.

13. The use of a compound or a composition according to Claim 12 wherein the soft drink is selected from tonic, orange soft drink, lemon soft drink, tea soft drink and a cola soft drink.

14. The use of a compound according to Claim 1 or a composition according to any of Claims 2 to 5 in the detection of y-hydroxybutyric acid (GHB) or a salt thereof in the urine.

15. A process for detecting GHB or a salt thereof in beverages, comprising:
a) taking a drop from the beverage to be analyzed and dissolving it in an aqueous solution of weak base;
b) adding an aliquot of the solution obtained in step a) to the Compound 1 or 2 according to claim 1 or to the composition according to any of claims 2 to 5;
c) observing the reaction produced; and
d) qualitatively determining the presence of GHB in the beverage to be analyzed according to the observation of step c).

16. A process for detecting GHB or a salt thereof in beverages, comprising:
a) mixing an aqueous solution of a weak base with an organic solvent;
b) adding compound 1 or 2 according to claim 1;
c) immediately after step b), adding a drop of the beverage to be analyzed;
d) observing the reaction produced; and
e) qualitatively determining the presence of GHB in the beverage to be analyzed according to the observation of step d).

17. The process according to claim 16, wherein the organic solvent is dimethylsulfoxide.

18. The process according to any of claims 15 to 17, wherein the weak base is sodium, potassium, ammonium or calcium bicarbonate.

19. The process according to any of claims 15 to 18, wherein the weak base is at a concentration between 0.4 mM and 1 mM.

20. A process for detecting GHB or a salt thereof in beverages comprising the steps of:
a) introducing a strip of non-woven fabric containing at least 50% polypropylene into a beverage sample;
b) introducing the beverage-impregnated strip of step a) into a container containing the composition according to any of claims 2 to 5, or alternatively Compound 1 or 2, according to claim 1;
c) observing the reaction produced in the container; and
d) qualitatively determining the presence of GHB in the beverage to be analyzed according to the observation of step c).

21. The process according to any one of claims 15 to 20, wherein compound 1 or 2 is at a concentration in the composition between 50 µM and 500 µM.

22. The process according to any of claims 15 to 21, wherein the beverage is an alcoholic beverage alone or mixed with a non-alcoholic beverage.

23. The process according to any of claims 15 to 21, wherein the beverage is a non-alcoholic beverage alone.

24. The process according to claim 22, wherein the alcoholic beverage is selected from gin, vodka, rum, whisky, white wine, vermouth and beer.

25. The process according to any of claims 22 to 24, wherein the non-alcoholic beverage is a soft drink or juice.

26. The process according to claim 25, wherein the soft drink is selected from tonic, orange soft drink, lemon soft drink, tea soft drink and a cola soft drink.

27. A process for detecting GHB or a salt thereof in urine, comprising:
a) preparing a urine sample from a subject by passing it through a chromatographic column, washing with methanol and water, and subsequently lyophillizing and redissolving the sample with water,
b) preparing a mixture of an aqueous solution of a weak base and the composition according to any of claims 2 to 5, or alternatively compound 1 or 2 according to claim 1;
c) mixing the urine sample obtained in step a) with the mixture of step b);
d) diluting the mixture obtained in step c) with an organic solvent;
e) observing the reaction produced; and
f) qualitatively and/or quantitatively determining the presence of GHB in the urine to be analyzed according to the observation of step e).

28. The process according to claim 27, wherein said weak base in step b) is sodium, potassium, ammonium or calcium bicarbonate.

29. The process according to any of claims 27 or 28, wherein the weak base is at a concentration between 0.4 mM and 1.2 mM.

30. The process according to any one of claims 27 to 29, wherein the compound 1 or 2 is at a concentration in the composition between 0.5 mM and 1.5 mM.

31. A kit or equipment for use in detecting GHB or a salt thereof in beverages and/or urine comprising
- a transparent container with opening and closure containing a compound 1 or 2 according to claim 1, or a composition according to any of claims 2 to 5, optionally wherein said container containing compound 1 or 2 is accompanied by another container with the same characteristics but with organic solvent;
- a transparent container with opening and closure containing a weak base in the form of an aqueous solution;
- a tool for collecting a sample of the beverage and/or urine to be analyzed

32. A kit or equipment for use in detecting GHB or a salt thereof in beverages comprising:
- a strip of non-woven fabric (NWF) with 50 to 100% polypropylene;
- a transparent container with opening and closure containing a composition according to any of claims 2 to 5, alternatively a compound 1 or 2 according to claim 1, optionally wherein said container containing compound 1 or 2 is accompanied by another container with the same characteristics but with organic solvent.

33. The kit or equipment according to claim 31 or 32 further comprising instructions for use.

34. The kit or equipment according to claim 31 or 33, wherein the weak base is sodium, potassium, ammonium or calcium bicarbonate.

35. The kit or equipment according to any of claims 31, 33 or 34, wherein the weak base is at a concentration between 0.4 mM and 1 mM when detecting beverages.

36. The kit or equipment according to any of claims 31, 33 or 34, wherein the weak base is at a concentration between 0.4 mM and 1.2 mM when detecting urine.

37. The kit or equipment according to any one of claims 31 to 35 wherein compound 1 or 2 is at a concentration in the composition between 50 µM and 500 µM when detecting beverages.

38. The kit or equipment according to any one of claims 31, 33, 34 and 36 wherein compound 1 or 2 is at a concentration in the composition between 0.5 mM and 1.5 mM when detecting urine.

39. The kit or equipment according to any of claims 31 to 35 and 37, wherein the beverage is an alcoholic beverage alone or mixed with a non-alcoholic beverage.

40. The kit or equipment according to any of claims 31 to 35 and 37, wherein the beverage is a non-alcoholic beverage.

41. The kit or equipment according to claim 39, wherein the alcoholic beverage is selected from gin, vodka, rum, whisky, white wine, vermouth and beer.

42. The kit or equipment according to any of claims 39 to 41, wherein the non-alcoholic beverage is a soft drink or juice.

43. The kit or equipment according to claim 42, wherein the soft drink is selected from among tonic, orange soft drink, lemon soft drink, tea soft drink, and a cola soft drink.
